# EUROPÄISCHE PATENTSCHRIFT

(11) **EP 2 106 258 B1**
(45) Veröffentlichungstag und Bekanntmachung des Hinweises auf die Patenterteilung: **12.09.2012**
(21) Anmeldenummer: 07818811.7
(22) Anmeldetag: 09.10.2007
(51) Int. Cl.: A61K 31/00, A61K 31/192, A61P 11/14

(54) **IBUPROFEN GEGEN HUSTEN**
IBUPROFEN AGAINST COUGHING
IBUPROFENE CONTRE LA TOUX

(30) Priorität: 20.10.2006 EP 06022042
(43) Veröffentlichungstag der Anmeldung: 07.10.2009
(73) Patentinhaber: Bayer Consumer Care AG, 4052 Basel (CH)
(72) Erfinder: PETERSEN-BRAUN, Marianne, 53639 Königswinter (DE); YEKRANGI-HARTMANN, Chirin, 40822 Mettmann (DE); GESSNER, Uwe, 51503 Rösrath (DE); VÖLKER, Michael, 50678 Köln (DE)
(74) Vertreter: Albers, Markus
(86) Internationale Anmeldenummer: PCT/EP2007/008737
(87) Internationale Veröffentlichungsnummer: WO 2008/046538

(56) Entgegenhaltungen:
- ANANIA A ET AL: "Anti-inflammatory drugs and bronchial reactivity" MINERVA PNEUMOLOGICA 1995 IT, Bd. 34, Nr. 3, 1995, Seiten 113-117, XP008097001 ISSN: 0026-4954
- PUJET J C ET AL: "Etude comparative de deux antitussifs dans le traitement des toux aiguees sèches d'origine infectieuse (étude prospective, randomisée, en simple aveugle)[Comparative study of two antitussive drugs in the treatment of acute dry cough of infectious origin (prospective, randomized, single blind study)." THERAPIE, DOIN, PARIS, FR, Bd. 57, Nr. 5, 1. September 2002 (2002-09-01), Seiten 457-463, XP008097000 ISSN: 0040-5957
- DALES R E ET AL: "Chronic cough responsive to ibuprofen" PHARMACOTHERAPY, BOSTON, US, Bd. 12, Nr. 4, 1. Januar 1992 (1992-01-01), Seiten 331-333, XP008096815 ISSN: 0277-0008 in der Anmeldung erwähnt
- TENENBAUM A ET AL: "Intermediate but not low doses of aspirin can suppress angiotensin-converting enzyme inhibitor-induced cough" AMERICAN JOURNAL OF HYPERTENSION, ELSEVIER, Bd. 13, Nr. 7, 1. Juli 2000 (2000-07-01), Seiten 776-782, XP008096998 ISSN: 0895-7061
- MCEWAN J R ET AL: "The effect of sulindac on the abnormal cough reflex associated with dry cough." THE JOURNAL OF PHARMACOLOGY AND EXPERIMENTAL THERAPEUTICS OCT 1990, Bd. 255, Nr. 1, Oktober 1990 (1990-10), Seiten 161-164, XP008097002 ISSN: 0022-3565

## Beschreibung

Die vorliegende Erfindung betrifft die Verwendung von Ibuprofen oder einem seiner pharmazeutisch annehmbaren Salze, Hydrate oder Derivate als Antitussivum bzw. ihre Verwendung zur Herstellung eines Medikaments für die Behandlung von unproduktivem Husten im Rahmen von viral oder bakteriell bedingten Erkrankungen der Atmungsorgane, insbesondere von Erkältungskrankheiten.

Ibuprofen ist ein Arylpropionsäurederivat, dessen schmerzlindernde (analgetische), fiebersenkende (antipyretische) und entzündungshemmende (antiinflammtorische) Wirkung seit langer Zeit bekannt ist. Es wird als Wirkstoff in einer Vielfalt von Arzneimitteln für eine orale, parenterale wie topische oder rektale Darreichung genutzt. Ibuprofenhaltige Präparate werden als Mittel zur Behandlung leichter bis mäßig starker Schmerzen wie Kopfschmerz, Zahnschmerz, Halsschmerz, Regelschmerz, Gelenkschmerz, Rückenschmerz, sowie bei der Behandlung der akuten Kopfschmerzphase bei Migräne mit und ohne Aura und der Behandlung von Spannungskopfschmerzen eingesetzt. Weitere Anwendungsgebiete für Ibuprofen sind die Behandlung entzündlicher und degenerativer Formen des Rheumatismus, Weichteilrheumatismus und nicht rheumatischer entzündlicher Schmerzzustände, von Arthrosen, entzündliche rheumatische Erkrankungen der Gelenke und der Wirbelsäule, Schwellungen bzw. Entzündungen der gelenknahen Weichteile, Schultersteife, Kreuzschmerzen, Hexenschuß, Sport- und Unfallverletzungen. Ibuprofenhaltige Präparate werden auch zur symptomatischen Behandlung von Fieberzuständen eingesetzt.

Ibuprofen gehört zu der Wirkstoffgruppe der nichtsteroidalen Antiphlogistika. Nichtsteroidale Antiphlogistika sind Inhibitoren der Cyclooxygenasen und in einer Reihe von unterschiedlichen oralen und parenteralen Darreichungsformen am Markt erhältlich. Für die topische Anwendung der nichtselektiven Cyclooxygenase-Inhibitoren gibt es Cremes und Gele. Ferner stehen als weitere parenterale Darreichungsformen Suppositorien und Injektionslösungen zur Verfügung. Parenterale Darreichungsformen für selektive Inhibitoren der Cyclooxygenase-2 sind zumindest kommerziell nicht erhältlich. Als oral zu verabreichende Darreichungsformen werden nichtsteroidale Antiphlogistika in Form von Tabletten, Filmtabletten, Retardtabletten, Dragees, Kapseln, Retardkapseln, Granulaten, Suspensionen und Brausetabletten angeboten.

Bekanntermaßen werden ibuprofenhaltige Präparate zur Behandlung der Symptome von viral bedingten Erkältungskrankheiten wie Fieber, Kopf-, Hals- und Gliederschmerz genutzt. Für die Behandlung des ebenfalls mit Erkältungskrankheiten vergesellschafteten Hustens werden dagegen andere Wirkstoffe aus der Gruppe der Expektorantien oder der Gruppe der Antitussiva wie Codein, Dihydrocodein, Hydrocodon, Clobutinol, Pentoxyverin, Pipazetat, Noscapin, Dextromethorphan oder pflanzliche Inhaltsstoffe verwendet.

Husten wird üblicherweise als Symptom einer Erkrankung angesehen, abgesehen vom idiopathischen Husten jedoch nicht als eigenständige Krankheit Husten (Tussis) bezeichnet das willkürliche oder aufgrund eines Hustenreizes über den Hustenreflex ausgelöste unwillkürliche, explosionsartige Ausstoßen von Luft, durch das die Atemwege von Fremdkörpern oder Schleim, die diese verlegen oder verengen könnten, befreit werden sollen. Bei Husten handelt es sich somit üblicherweise um einen körpereigenen Schutz- und Reinigungsmechanismus.

Es werden verschiedene Hustenarten unterschieden. Im wesentlichen wird zwischen trockenem Husten (unproduktiv) und dem durch reichliche Schleimbildung gekennzeichneten produktiven Husten differenziert. Ferner unterscheidet man nach dem zeitlichen Verlauf akuten und chronischen Husten.

Der trockene Husten (Reizhusten) zeichnet sich durch krampfartige, harte Hustenattacken aus, bei denen kein Schleim ausgeworfen wird. Er tritt plötzlich auf und ist in der Regel nur von kurzer Dauer, kann aber auch über mehrere Wochen andauern. Beim Reizhusten führt das Husten selbst nicht zu einer Verringerung des Hustenreizes.

Produktiver Husten tritt auf, wenn das Bronchialsekret (Schleim) in den Atemwegen durch die Reinigungskraft der Flimmerhärchen nicht mehr entfernt werden kann. Dann soll das Sekret durch den Husten gelöst und abtransportiert werden. Der Hustenreiz ist beim produktiven Husten meist sehr stark, läßt nach Auswurf des Bronchialsekrets jedoch nach.

Beim "Raucherhusten", der auch als chronischer produktiver Husten bezeichnet wird und ebenso bei Nichtrauchern auftreten kann, kommt es aufgrund einer dauerhaften Schädigung der Flimmerhärchen infolge einer langfristigen Reizung der Bronchien zu einer Vergrößerung der Drüsen in der Bronchialschleimhaut sowie zu einer vermehrten Schleimproduktion. Anstelle des natürlichen Selbstreinigungsmechanismus tritt ein starker Hustenreiz auf, um den Schleim abzutransportieren. Dieser Husten tritt vermehrt in den frühen Morgenstunden auf und ist ein Krankheitszeichen für eine chronische Bronchitis.

Der Erkältungshusten ist ein häufiges Symptom bei grippalen Infekten, d.h. bei fast immer durch Viren verursachten Infektionen der Atmungsorgane. Kurz nachdem sich die Viren in den Bronchien festgesetzt haben, entwickelt sich aufgrund der Schleimhautreizung ein trockener Reizhusten, der in einen schleimlösenden produktiven Husten übergeht. Ist der schleimige Auswurf gelblich, grünlich oder bräunlich verfärbt, ist dies ein Zeichen für eine bakterielle Infektion. Wenn die Erkältung abklingt, gehen die Symptome zurück und der Husten geht wieder in einen trockenen Husten über und/oder verschwindet.

Erkältungskrankheiten bzw. die sogenannten "grippalen Infekte", die üblicherweise auf eine Infektion mit Viren zurückzuführen sind, gelten als häufigste Ursache für Husten.

Als Beispiel für bakteriell bedingten unproduktiven Husten ist insbesondere Keuchhusten zu nennen. Keuchhusten ist eine schwere Infektionskrankheit, die durch das Bakterium *Bordetella pertussis* verursacht wird.

Keuchhusten läßt sich in drei Stadien unterteilen. Er beginnt etwa fünf bis vierzehn Tage nach der Ansteckung mit dem so genannten "Stadium catarrhale", das ein bis zwei Wochen dauert. In diesem Stadium leidet der Infizierte unter grippeähnlichen Symptomen wie Niesen, laufender Nase, rauhem Hals, leichtem Husten und mäßigem Fieber. Darauf folgt das für diese Krankheit charakteristische Stadium (Stadium convulsivum), das etwa vier bis sechs Wochen andauert. Der Husten verschlimmert sich und tritt krampfartig in Form mehrerer aufeinander folgender starker Hustenanfälle auf, gefolgt von einem plötzlichen laut keuchenden Einatmen. Bei diesen Hustenanfällen kann Schleim hervorgewürgt und anschließend erbrochen werden. Die Hustenattacken treten gehäuft nachts auf und können durch äußere Anlässe wie zum Beispiel körperliche Anstrengung oder psychische Faktoren ausgelöst werden. Zwischen den Hustenanfällen sind die Betroffenen in der Regel unbeeinträchtigt von der Krankheit und leiden nicht unter Fieber. Im Spätstadium der Erkrankung, dem sogenannten "Stadium decrementi", klingen die Hustenstöße allmählich ab. Diese Phase unproduktiven Hustens dauert ohne antibiotische Behandlung nach Abklingen der fiebrigen Erkrankungsphase meist noch sechs bis zehn Wochen.

Als Ursachen für Husten kommen aber nicht nur Erkrankungen der Atmungsorgane in Frage, sondern es können auch Erkrankungen des Herzens oder des Magens sein. Darüber hinaus kann dem Husten aber auch die Einnahme von Medikamenten oder in seltenen Fällen eine psychische Störung zugrunde liegen.

Zur Behandlung von Husten werden entweder den Auswurf von Bronchialsekret fördernde Medikamente (Expektorantien) oder den Hustenreiz unterdrückende Medikamente (Antitussiva) verabreicht.

Bei den Expektorantien unterscheidet man je nach Wirkungsmechanismus zwischen den Sekretolytika bzw. Mukolytika, die eine Verflüssigung des Bronchialsekrets erzielen, und den Sekretomotorika, durch die ein verstärkter Abtransport des Bronchialschleims ausgelöst wird.

Als Sekretolytika gelten beispielsweise Acetylcystein, Bromhexin und dessen Metabolit Ambroxol, Ammoniumchlorid und Guaifenesin, aber auch pflanzliche Produkte wie Fenchel- und Anisöl.

Bei den meisten Antitussiva handelt es sich um Opiatabkömmlinge und damit verschreibungspflichtige Medikamente. Verwendet werden beispielsweise Codein, Dihydrocodein, Dextromethorphan oder Hydrocodon, das besonders stark wirksam ist und unter das Betäubungsmittelgesetz fällt. Diese Substanzen wirken über das Zentralnervensystem und haben neben einem dämpfenden Effekt auf das Hustenzentrum im Stammhirn auch einen beruhigenden (sedierenden) Effekt.

Alternativ sind Substanzen einsetzbar, die kein Suchtpotential haben und auch nicht sedierend wirken. Zu dieser Wirkstoffgruppe gehören Clobutinol und das etwas schwächer wirksame Pentoxyverin.

Alternativ kann auch die Einnahme bestimmter pflanzlicher Präparate eine Linderung des Hustenreizes herbeiführen.

Hustenreiz unterdrückende Medikamente sollten nur bei unproduktivem Husten gegeben werden. Eine Kombination mit schleimlösenden Mitteln (Expektorantien) ist kontraproduktiv, da der entstehende Schleim dann nicht abgehustet werden kann.

Die Verwendung von Ibuprofen in Kombination mit einem Antitussivum zur Behandlung des mit Erkältungskrankheiten einhergehenden Hustens ist nicht nur gebräuchlich und in zahlreichen Veröffentlichungen beschrieben, sondern auch Gegenstand zahlreicher Patent- und Offenlegungsschriften. Beispielsweise offenbart EP 0 274 845 A1 eine stabile, feste pharmazeutische Zubereitung, die Ibuprofen oder ein pharmazeutisch annehmbares Salz des Ibuprofens in Kombination mit Codein oder einem pharmazeutisch annehmbaren Salz des Codeins und ein unlösliches Salz der Carboxymethylcellulose in einer eine Entfärbung der Zubereitung verhindernden Menge enthält.

Bei den bekannten Wirkstoffkombinationen mit einem Antitussivum wird Ibuprofen jedoch ausschließlich als Analgetikum oder als Antipyretikum zusammen mit dem Antitussivum eingesetzt.

Über die Behandlung von Husten mit analgetisch wirksamen Monopräparaten liegen dagegen nur wenige Literaturberichte vor. So wurde von Ulukol et al. (Eur. J. Clin. Pharmacol. 1999; 55: 615-618) die Verringerung des Symptoms Husten nach der Behandlung mit Ibuprofen in einer Dosierung von 10 mg/kg in einer Studie an 30 Kindern beschrieben, die unter einer fiebrigen Infektion der oberen Atemwegorgane litten.

Im Rahmen dieser Studie wurden Kinder mit einer fiebrigen Erkältung der oberen Atemwegsorgane über 5 Tage hinweg entweder mit Paracetamol (3 X täglich 10 mg/kg), mit Ibuprofen (3 X täglich 10 mg/kg) oder mit Nimesulid (2 X täglich 2,5 mg/kg) behandelt. Hierbei stellte sich heraus, daß die fiebersenkende Wirkung von Nimesulid besser und schneller als die von Paracetamol oder Ibuprofen war. Eine Linderung der Hustensymptomatik war bei den mit Paracetamol behandelten Kindern stärker als bei den Kindern, die einen der beiden anderen Wirkstoffe erhielten.

Allerdings handelte es sich bei dieser Studie weder um eine doppelblinde Studie, noch umfaßte sie eine Placebogruppe als Kontrolle. Sie ist daher nicht als kontrollierte Studie anzusehen. Da Erkältungskrankheiten zudem selbstlimitierende Erkrankungen sind, kann eine dem Ibuprofen zuzuschreibende hustenreizstillende Wirkung aus dieser Studie nicht abgeleitet werden.

In einer Fallbeobachtung an einer 57-jährigen Frau mit idiopathischem chronischem Husten, d.h. einem Husten unbekannter Ursache, wurde unter Therapie mit Ibuprofen in einer Dosierung von dreimal täglich 600 mg für 6 Tage eine Verringerung der Anzahl der Hustenanfälle im Vergleich zum Placebo festgestellt (Dales et al., Pharmacotherapy 1992; 12: 331-333). Es ist jedoch bemerkenswert, daß die verabreichte Ibuprofenmenge mit einer täglichen Dosis von 1800 mg wesentlich über der für den OTC-Bereich (OTC = "over-the-counter") vorgesehenen Dosis von 400 bis maximal 1200 mg/Tag lag.

Die Verabreichung von Ibuprofen als Monopräparat zur Behandlung des Hustens als Symptom viral oder bakteriell bedingter Erkrankungen der Atmungsorgane, insbesondere von Erkältungszuständen, ist jedoch nicht bekannt. Im Stand der Technik wird dieser mit clobutinol behandelt, siehe Pujet et al., Thérapie 2002;57; 457- 463.

Anania et al. (Minerva Pneumologia 1995, Vol. 34, S. 113-117) beschreibt, dass mit Nimesulid ein nichtsteroidales Antiphlogistikum als hustenstillendes Mittel bei Erkrankungen eingesetzt werden kann, bei denen "entzündliche Prozesse eine dominierende Rolle" spielen, allerdings wird dort ebenfalls offenbart, dass sich die Wirkung von Nimesulid in dieser Hinsicht deutlich von denen anderer nichtsteroidaler Antiphlogistika unterscheide.

Gegenstand der vorliegenden Erfindung ist die Verwendung von Ibuprofen oder einem seiner pharmazeutisch annehmbaren Salze oder Hydrate zur Behandlung viral oder bakteriell bedingten unproduktiven Hustens, bzw. zur Herstellung eines Medikaments für die Behandlung von viral oder bakteriell bedingtem unproduktivem Husten, insbesondere in der Phase der Erkrankung, in der der Reizhusten im Abklingen begriffen ist.

In einer besonders bevorzugten Ausführongsform der Erfindung wird als Arzneimittel-Wirkstoff Ibuprofen zur Herstellung des Medikaments zur Behandlung eines viral oder bakteriell bedingten Hustens verwendet. Dabei kann das Ibuprofen sowohl in Form eines racemischen Gemisches seiner beiden Enantiomere (R-Ibuprofen und S-Ibuprofen) verwendet werden, als auch in Form eines seiner beiden Enantiomere, dem pharmakologisch wirksamen S-Ibuprofen oder dem R-Ibuprofen, welches in vivo in das pharmakologisch wirksame S-Ibuprofen umgewandelt wird, sowie einem Salz oder Hydrat davon.

In einer bevorzugten Ausführungsform handelt es sich bei dem Medikament zur Behandlung des viral oder bakteriell bedingten unproduktiven Hustens um ein Monopräparat, d.h. um ein Medikament, das Ibuprofen oder ein pharmazeutisch annehmbares Salz des Ibuprofens als einzigen Arzneimittel-Wirkstoff enthält.

Die erfindungsgemäßen Verbindungen können auch in Form ihrer Salze, Hydrate und/oder Solvate vorliegen.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Diese Mischungen der Enantiomere und Diastereomere lassen sich in bekannter Weise in die stereoisomer einheitlichen Bestandteile trennen.

Als Salze sind im Rahmen der Erfindung physiologisch unbedenkliche Salze der erfindungsgemäßen Verbindungen bevorzugt.

Physiologisch unbedenkliche Salze der erfindungsgemäßen Verbindungen können Säureadditionssalze der Verbindungen mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ehansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können aber auch Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabiethylamin, 1-Ephenamin oder Methyl-piperidin.

Hydrate der erfindungsgemäßen Verbindungen sind im Rahmen der Erfindung stöchiometrische Zusammensetzungen der Verbindungen oder seinen Salzen mit Wasser.

Solvate der erfindungsgemäßen Verbindungen sind im Rahmen der Erfindung stöchiometrische Zusammensetzungen der Verbindungen oder seinen Salzen mit Lösungsmittel.

Der besondere Vorteil dieses Monopräparats besteht darin, dass das Ibuprofen bei einer viral bedingten Erkältungskrankheit neben dem Symptom Husten auch die weiteren üblichen Symptome Fieber und Schmerzen (Kopf-, Glieder- und Muskelschmerzen) lindert, ohne daß eine Einnahme weiterer Medikamente erforderlich ist.

**Tabelle 1: Wirkstoffmengen, die als Einheitsdosis in Formulierungen unterschiedlicher nichtsteroidaler Antiphlogistika enthalten sind.**

| Wirkstoff | Wirkstoffmenge [mg] |
|---|---|
| Ibuprofen | 200, 400, 600, 800 |
| Ketoprofen | 50, 100, 200 |
| Naproxen | 220, 250, 500, 660, 750 |
| Diclofenac | 12,5, 25, 50, 100 |
| Celecoxib | 100, 200 |
| Rofecoxib | 12,5, 25 |
| Valdecoxib | 10,20,40 |

Die gemäß der erfindungsgemäßen Verwendung erhältlichen Medikamente können Ibuprofen in üblichen Dosierungen enthalten, die von dem verwendeten Arzneimittel-Wirkstoff abhängig sind. Beispiele für übliche Wirkstoffmengen, die als Einheitsdosen in Formulierungen von nichtsteroidalen Antiphlogistika enthalten sind, werden in Tabelle 1 aufgeführt. Insbesondere bei Verwendung von Ibuprofen, oder einem seiner pharmazeutisch annehmbaren Salze oder Hydrate kann das Medikament eine Dosis von 50 bis 3000 mg, vorzugsweise von 100 bis 800 mg und besonders bevorzugt von 200 bis 400 mg Ibuprofen äquivalente Menge Wirkstoff enthalten. Gegebenenfalls kann es erforderlich sein, von den genannten Mengen abzuweichen, und zwar in Abhängigkeit von Körpergewicht bzw. der Art des Applikationsweges, vom individuellen Verhalten gegenüber den Medikamenten, der Art von deren Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Die erfindungsgemäßen Medikamente und pharmazeutischen Formulierungen umfassen sowohl parenteral als auch oral verabreichbare Darreichungsformen, wobei Darreichungsformen zur oralen Verabreichung bevorzugt werden.

Bei den erfindungsgemäßen Medikamenten bzw. pharmazeutischen Formulierungen kann es sich um flüssige oder feste Darreichungsformen handeln. Beispiele für feste Darreichungsformen sind Tabletten, Schmelztabletten, Brausetabletten, Dragees, Kapseln, Weichgelatinekapseln, Pulver, Granulate, Pellets, Suppositorien sowie Plättchen und Oblaten. Beispiele für flüssige Darreichungsformen sind Säfte, Sirupe, Gele, vorzugsweise niedrig viskose Gele, Suspensionen und Lösungen.

Die Wirkstoffe der erfindungsgemäßen Medikamente und pharmazeutischen Formulierungen sind besonders geeignet, in einer fixen Kombination in Form einer festen peroralen Darreichungsform formuliert zu werden. Es ist bekannt, daß die Einnahmezuverlässigkeit (Compliance) bei Patienten entscheidend von den Faktoren Anzahl der Darreichungsformen pro Einnahmezeitpunkt, sowie Größe und Gewicht der (festen peroralen) Arzneiform abhängig ist. Daher sollte sowohl die Anzahl der verschiedenen getrennt einzunehmenden Arzneimittel so gering wie möglich sein (Vorteil einer fixen Kombination), als auch die Größe und das Gewicht einer festen peroralen Darreichungsform so klein wie möglich gehalten werden, ohne die therapeutische Wirkstärke zu beeinträchtigen. Dadurch wird die Einnahme für den Patienten so angenehm wie möglich gestaltet. Durch Realisieren fixer Kombinationen in Form von festen peroralen Arzneiformulierungen mit minimaler Größe und minimalem Gewicht wird eine höchstmögliche Patienten-Compliance geboten und die Sicherheit sowie Zuverlässigkeit der Therapie entscheidend verbessert.

Durch Modifikation der Zusammensetzung der Medikamente oder pharmazeutischen Formulierungen kann die Wirkstofffreisetzung aus dem Medikament gesteuert werden. Beispielsweise läßt sich durch verzögerte Freisetzung der oder eines der Wirkstoffe in einer fixen Kombination deren Wirkungseintritt auch bei Verwendung von Fixkombinationen zeitlich entkoppeln.

Die Herstellung der erfindungsgemäßen Medikamente und pharmazeutischen Formulierungen zur Behandlung des unproduktiven Hustens kann unter Berücksichtigung der erfindungsgemäß zu verwendenden nichtsteroidalen Antiphlogistika mit den für die jeweilige Darreichungsform bekannten Verfahren unter Verwendung von pharmazeutischen Hilfsstoffen, wie sie dem Fachmann geläufig sind, erfolgen.

Die festen Darreichungsformen werden nach den allgemein bekannten Standardverfahren hergestellt. Weitere Inhaltsstoffe sind solche, die pharmakologisch annehmbar und physiologisch unbedenklich sind, beispielsweise: als Füllstoffe Cellulosederivate (z.B. mikrokristalline Cellulose), Zucker (z.B. Lactose), Zuckeralkohole (z.B. Sorbitol, Mannitol), anorganische Füllstoffe (z.B. Calciumphosphate), Bindemittel (z.B: Polyvinylpyrrolidon, Gelatine, Stärkederivate und Cellulosederivate), sowie alle weitere Hilfsstoffe, die zur Herstellung von Arzneiformulierungen der gewünschten Eigenschaften benötigt werden, z.B. Schmiermittel (Magnesiumstearat), Sprengmittel (z.B. quervernetztes Polyvinylpyrrolidon, Natriumcarboxymethylcellulose), Netzmittel (z.B. Natriumlaurylsulfat), Retardierungsmittel (z.B. Cellulosederivate, Poly(meth)acrylsäurederivate), Stabilisatoren, Aromen und/oder Farbpigmente.

Flüssige Darreichungsformen werden ebenfalls nach Standardmethoden mit pharmazeutisch annehmbaren Hilfsstoffen hergestellt und enthalten die Wirkstoffe entweder gelöst oder suspendiert. Typische Applikationsvolumina dieser Arzneizubereitungen liegen zwischen 1 und 10 ml. Beispiele für Hilfsstoffe in diesen flüssigen Formulierungen sind: Lösungsmittel (z.B. Wasser, Alkohol, natürliche und synthetische Öle wie mittelkettige Triglyceride), Lösungsvermittler (z.B. Glycerol, Glykolderivate), Netzmittel (z.B. Polysorbat, Natriumlaurylsulfat), sowie weitere Hilfsstoffe, die zur Herstellung von Arzneiformulierungen der gewünschten Eigenschaften benötigt werden, z.B. viskositätserhöhende Mittel, pH-Wert-Korrigenzien, Süßstoffe und Aromen, Antioxidantien, Stabilisatoren und/oder Konservierungsmittel.

Hauptbestandteil der Hüllen von Kapselformulierungen sind beispielsweise Gelatine oder Hydroxypropylmethylcellulose.

### Beispiel:

Bei 3 freiwilligen Patienten wurde in der Endphase eines viralen Infekts die Wirksamkeit von Ibuprofen bei der symptomatischen Behandlung des nichtproduktiven Hustens festgestellt. Dazu wurden den Patienten 400 oder 800 mg Ibuprofen peroral verabreicht (die Tagesdosis betrug zwischen 400 und 1200 mg). Andere Medikamente wurden von den Patienten während des viralen Infekts nicht eingenommen.

Nach Gabe von Ibuprofen wurde beobachtet, daß die Anzahl der Hustenattacken bezogen auf die Anzahl der Hustenattacken vor Gabe von Ibuprofen, um mehr als 50% reduziert war. Die Patienten äußerten übereinstimmend, daß auch die Intensität des Hustenreizes und die Schwere der Hustenattacken reduziert waren. Im Zusammenhang damit konnte auch die Nachtruhe der Patienten verbessert werden, ohne daß die Gabe eines zusätzlichen Hustenstillers erforderlich war.

Nach 6 bis 8 Stunden klangen die Ibuprofen-Wirkungen ab und der Husten trat in der alten Stärke wieder auf. Sich daran anschließende erneute Gaben von Ibuprofen führten wieder zu der beschriebenen Verbesserung der Hustensymptomatik.

## Patentansprüche

1. Verwendung von Ibuprofen oder einem seiner pharmazeutisch annehmbaren Salz oder Hydrate zur Herstellung eines Medikaments zur Behandlung von unproduktivem Husten als Symptom viral und/oder bakteriell bedingter Infektionen der Atmungsorgane.

2. Verwendung nach Anspruch 1, **dadurch gekennzeichnet, dass** es sich bei Ibuprofen um ein racemisches Gemisch aus R-Ibuprofen und S-Ibuprofen handelt.

3. Verwendung nach Anspruch 1, **dadurch gekennzeichnet, dass** es sich bei Ibuprofen um das S-Ibuprofen handelt.

4. Verwendung nach Anspruch 1, **dadurch gekennzeichnet, dass** es sich bei Ibuprofen um R-Ibuprofen handelt.

5. Verwendung nach einem der vorangehenden Ansprüche, dadurch gekenntzeichnet, dass sie mit einem das Ibuprofen als einzigen Arzneimittelwirkstoff enthaltenden Monopräparat erfolgt.

6. Ibuprofen oder eines seiner pharmazeutisch annehmbaren Salze oder Hydrate zur Verwendung in der Behandlung von unproduktivem Husten als Symptom viral und/oder bakteriell bedingter Infektionen der Atmungsorgane.

## Claims

1. Use of ibuprofen or one of its pharmaceutically acceptable salts or hydrates for the manufacture of a medicament for the treatment of nonproductive cough as symptom of infections of the respiratory organs caused by viruses and/or bacteria.

2. Use according to Claim 1, **characterized in that** ibuprofen is in the form of a racemic mixture of R-ibuprofen and S-ibuprofen.

3. Use according to Claim 1, **characterized in that** ibuprofen is in the form of S-ibuprofen.

4. Use according to Claim 1, **characterized in that** ibuprofen is in the form of R-ibuprofen.

5. Use according to any of the preceding claims, **characterized in that** it takes place with a single-drug product comprising the ibuprofen as sole active pharmaceutical ingredient.

6. Ibuprofen or one of its pharmaceutically acceptable salts or hydrates for use in the treatment of nonproductive cough as symptom of infections of the respiratory organs caused by viruses and/or bacteria.

## Revendications

1. Utilisation d'ibuprofène ou d'un de ses sels ou hydrates pharmaceutiquement acceptables pour la fabrication d'un médicament pour le traitement de la toux non productive en tant que symptôme d'infections d'origine virale et/ou bactérienne des organes respiratoires.

2. Utilisation selon la revendication 1, **caractérisée en ce que** l'ibuprofène est un mélange racémique d'ibuprofène R et d'ibuprofène S.

3. Utilisation selon la revendication 1, **caractérisée en ce que** l'ibuprofène est l'ibuprofène S.

4. Utilisation selon la revendication 1, **caractérisée en ce que** l'ibuprofène est l'ibuprofène R.

5. Utilisation selon l'une quelconque des revendications précédentes, **caractérisée en ce qu'**elle a lieu avec une monopréparation contenant l'ibuprofène en tant qu'agent actif médicamenteux unique.

6. Ibuprofène ou un de ses sels ou hydrates pharmaceutiquement acceptables destiné à être utilisé pour le traitement de la toux non productive en tant que symptôme d'infections d'origine virale et/ou bactérienne des organes respiratoires.
